(19) Europäisches Patentamt
European Patent Office
Office européen des brevets

(11) Veröffentlichungsnummer: **0 266 548 B1**

(12) # EUROPÄISCHE PATENTSCHRIFT

(45) Veröffentlichungstag der Patentschrift: **04.09.91**

(51) Int. Cl.5: **C09J 201/02**, C09J 5/00, A61K 6/08, //C08F291/00, C08F220/36,C08G59/14, C08L63/10,C08L101/00

(21) Anmeldenummer: **87114165.1**

(22) Anmeldetag: **29.09.87**

(54) **Verwendung polymerisierbarer Mischungen zur Herstellung adhäsiver Produkte für biologische Substrate basierend auf Verbindungen, die aus Aldehyd-, Epoxid-, Isocyanat- oder Halotriazingruppen, polymerisierbaren Gruppen und einem höhermolekularen Grundgerüst bestehen.**

(30) Priorität: **08.10.86 DE 3634354**

(43) Veröffentlichungstag der Anmeldung:
**11.05.88 Patentblatt 88/19**

(45) Bekanntmachung des Hinweises auf die Patenterteilung:
**04.09.91 Patentblatt 91/36**

(84) Benannte Vertragsstaaten:
**CH DE FR GB IT LI**

(56) Entgegenhaltungen:
**EP-A- 0 124 812**
**EP-A- 0 126 359**
**EP-A- 0 142 747**
**DE-A- 1 901 986**
**DE-A- 2 151 648**
**DE-B- 2 140 404**
**FR-A- 2 213 306**
**US-A- 4 595 734**

**H.JAHN "Epoxidharze",1969, VEB Deutscher Verlag für Grundstoffindustrie, Leipzig, Seite 27**

(73) Patentinhaber: **Ernst Mühlbauer KG**
**Fangdieckstrasse 61**
**W-2000 Hamburg 53(DE)**

(72) Erfinder: **Engelbrecht, Jürgen, Dr. Dipl.-Chem.**
**Petkumstrasse 18**
**W-2000 Hamburg 76(DE)**

(74) Vertreter: **Beil, Hans Christoph, Dr. et al**
**Beil, Wolff und Beil Rechtsanwälte Adelonstrasse 58**
**W-6230 Frankfurt am Main 80(DE)**

**EP 0 266 548 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von polymerisierbaren Mischungen Zur Herstellung eines Adhäsivs für biologische Substrate, die als haftvermittelnde Komponente oligomere oder prepolymere Verbindungen enthalten, die neben mehreren polymerisierbaren ungesättigten Gruppen als funktionelle Haftgruppen ausschließlich noch mehrere Aldehyd-, Epoxid-, Isocyanat- oder Halotriazingruppen an ein oligomeres oder polymeres Grundgerüst gebunden enthalten. Derartige Mischungen haften auf verschiedenen biologischen Substraten. Mischungen dieser Art können für allgemeine Zwecke, aber besonders im dentalen Bereich als polymerisierbare Haftvermittler, Haftfüllungsmaterialien und Haftzemente eingesetzt werden.

Polymerisierbare Mischungen auf der Basis von Monomeren mit einer oder mehreren ungesättigten Gruppen versehenen Verbindungen bilden die Grundlage für eine Vielzahl von Kunststoffen, wobei im dentalen und medizinischen Bereich hauptsächlich Verbindungen mit Methacrylatgruppen wichtig sind. Mischungen dieser Art sind z.B. die Grundlage für Kunststoffüllungs- und Versiegelungsmaterialien.

Diese polymerisierbaren Mischungen können jedoch im allgemeinen keine chemischen Verbindungen mit anderen Materialien, besonders biologischen Substraten, ausbilden, es sei denn, diese Substrate enthalten noch selbst ausreichend unpolymerisierte polymerisierbare Gruppen.

Eine festere Verbindung kann daher nur über retentionsreiche Flächen, d.h. über einen Verbund rein mechanischer Art, z.B. durch Anätzen der Oberfläche von biologischen oder anorganischen Materialien erreicht werden. Durch Anwendung von Haftvermittlern, d.h. von Substraten, die mit biologischem oder anorganischem Material chemisch reagieren können als auch andererseits eine polymerisierbare Gruppe tragen, kann dieser Mangel jedoch behoben werden.

Es ist eine Reihe von solchen Haftvermittlern bekannt, z.B. die Vinyl- oder Methacrylgruppen tragenden Organosilane. Sie sind jedoch in ihrer Haftwirkung auf Siliciumdioxid, Siliciumdioxid enthaltende Gläser und Keramiken und Metalloxide, bzw. diese bildende Unedelmetalle beschränkt. Auf biologischen Substraten und besonders auf Zahn- oder Knochensubstrat zeigen sie keine Haftung, sondern wirken eher trennend.

Für Substrate solcher Art sind eine Reihe von polymerisierbaren Haftvermittlern mit anderen Haftgruppen gefunden worden. Da sind solche, die zum einen vorzugsweise mit dem Collagen oder collagenähnlichen Anteilen dieser Substrate reagieren wie z.B. Benzaldehydmethacrylat ( J.M.Antonucci, J.Dent. Res. 57, 500 (1978) ), Kombinationen von Dialdehyden mit Hydroxyalkylmethacrylaten (EP-0141324), Epoxyalkylmethacrylate, Isocyanoalkylmethacrylate sowie Kombinationen von Diisocyanaten mit Hydroxy- oder Amino-Gruppen enthaltenden Methacrylaten sowie Allylhalotriazine ( USP 4,203,220). Ferner gibt es eine Zahl von polymerisierbaren Verbindungen, die mit den Apatit-Verbindungen der Zahn- oder Knochensubstanz reagieren. Diese haftvermittelnden Verbindungen tragen Säuregruppen oder reaktive Säuregruppenderivate. Beispiele solcher polymerisierbarer Verbindungen sind:

ungesättigte organische Ester von Phosphor- oder Phosphonsäuren
(DE-AS 27 11 234, DE-OS 31 50 285);
ungesättigte organische Ester der Monofluorphosphatsäure
(US-PS 3 997 504);
ungesättigte organische Ester von Säuren des Phosphors, die Chlor oder Brom direkt am Phospor gebunden enthalten
(EP 0 058 483);
ungesättigte organische Ester der Phosphorsäure, die als cyclische Pyrophosphate (Anhydride) vorliegen
(DE-OS 30 48 410);
ungesättigte Carbonsäuren und reaktive Cabonsäurederivate,wie 4-Methacryloyloxyethyltrimellitsäure und deren Anhydrid (Takeyama, M. et al.. I. Jap. Soc. f. Dent. App. a. Mat. 19, 179 (1978) oder
Pyromellit säure-bis-2-methycryloylethylester.

Durch die aufgeführten polymerisierbaren Verbindungen ist in vielen Fällen eine mehr oder weniger starke Haftung von polymerisierbaren Füllungs- und Versiegelungsmaterialien an Zahn-und Knochensubstanz zu erreichen. Der Erfolg der Anwendung hängt auch weitgehend davon ab, in welcher Schichtdicke die haftvermittelnde Substanz aufgetragen wird, wie viele Haftgruppen mit dem biologischen Substrat und wie viele polymerisierbare Gruppen mit dem zu copolymerisierenden Material zur Reaktion kommen.

In der deutschen Patentanmeldung DE-A-35 36 077 (Engelbrecht et al.) wurden oligomere und prepolymere Verbindungen beschrieben, die an ein oligomeres oder prepolymeres Grundgerüst eine Vielzahl von polymerisierbaren Gruppen und eine Vielzahl von Haftgruppen gebunden enthalten. Dabei sind die Haftgruppen Säuregruppen oder deren reaktive Derivate.

Solche Verbindungen tragen eine Vielzahl von polymerisierbaren Gruppen und Haftgruppen und zeigen daher eine erhebliche höhere Haftkraft als entsprechende monomere Verbindungen.

Ein zusätzlicher Einbau von Aldehyd-, Epoxid, Isocyanat- oder Halotriazingruppen in diese säuregruppenhaltigen polymerisierbaren Oligomeren oder Prepolymeren konnte die Haftkraft besonders am Dentin in einigen Fällen nochmals erhöhen. Diese Gruppen, die besonders für eine zusätzliche Bindung zu collagenähnlichen Substraten in die höhermolekularen Verbindungen eingeführt sind, sind jedoch nicht in allen Fällen mit den Säuregruppen oder deren reaktiven Derivaten verträglich, zudem blockieren die sauren Gruppen eine Reihe von aminischen Polymerisationsinitiatoren.

In der Druckschrift H. Jahn "Epoxidharz", 1969, s. 27 wird ein epoxidiertes Butadienoligomeres offenbart, jedoch keine Eigenschaften und Verwendungsmöglichkeiten derselben.

Die DE-A 1 901 986 betrifft ausgehärtete polymere Verbindungen der obengenannten Art, welche als Isolatoren, Überzüge, Dichtungen und dergleichen verwendet werden können.

Die US-PS 4 595 734 beschreibt ein Novolak-Epoxidharz, abgeleitet von z.B. Bisphenol A und Epichlorhydrin, welches ein verbessertes Viskositätsverhalten aufweist.

Die EP-A 0 126 359 beschreibt ein Isophorondiisocyanat, welches mit Hydroxypropylmethacrylat zu einem Oligomer mit freien NCO-Gruppen sowie freien olefinischen (polymerisierbaren) Doppelbindungen umgesetzt wurde. Dieses Produkt weist eine gute Haftfähigkeit auf technischen Stoffen, wie z.B. Aluminiumblech, bzw. eine gute Eignung für Verpackungszwecke und zur Isolation auf.

Die DE-B 2 140 404 beschreibt polymerisierte Produkte, die noch Isocyanat enthalten und in einem Lösungsmittel hergestellt werden und als solche oder aus der Schmelze als Zahnlack eingesetzt werden.

Der Erfindung lag daher die Aufgabe zugrunde, polymerisierbare Mischungen zur Herstellung von Adhäsivprodukten für biologische Substrate zu finden, die als Haftvermittler solche Verbindungen enthalten, die als funktionelle Haftgruppen ausschließlich eine Vielzahl von Aldehyd-, Epoxid-, Isocyanat- oder Halotriazingruppen und eine Vielzahl von polymerisierbaren Gruppen, jedoch keine Säuregruppen oder deren reaktive Derivate tragen. Erfindungsgemäß wird diese Aufgabe gelöst durch polymerisierbare Mischungen, die als Haftvermittler oligomere oder prepolymere organische Verbindungen enthalten, die aus chemisch recht stabilen molekularen Grundgerüsten, mehreren polymerisierbaren Gruppen und mehreren Aldehyd-, Epoxid-, Isocyanat- oder Halotriazingruppen bestehen.

Es wurde gefunden, daß für diesen Zweck solche Mischungen geeignet sind, die als Haftvermittler oligomere oder polymere Grundgerüste aus Homo- oder Co-Oligomerisaten bzw. Homo- oder Co-Polymerisaten enthalten, die die gewünschten Aldehyd-, Epoxid-, Isocyanat oder Halotriazingruppen enthalten und an die polymerisierbare Gruppen aufgepfropft werden können, oder solche, die funktionelle Gruppen enthalten, an die sowohl die gewünschten Aldehyd-, Epoxid-, Isocyanat- oder Halotriazingruppen, als auch die polymerisierbaren Gruppen geknüpft werden können. Vorteilhafterweise enthalten die Haftverbindungen der erfindungsgemäß verwendeten Mischungen drei oder mehrere polymerisierbare ungesättigte Gruppen und drei oder mehrere Aldehyd-, Epoxid-, Isocyanat- oder Halotriazingruppen.

Dabei kann je nach Substrat, auf dem die Verbindungen haften sollen, oder je nach polymerisierbarer Mischung, in der sie enthalten sind, die Zahl der polymerisierbaren Gruppen oder die Zahl der Haftgruppen überwiegend oder gleich sein.

Als polymerisierbare ungesättigte Gruppen dieser Haftvermittler eignen sich alle Alkenyl-, Alkenoxy-, Cycloalkenyl-, Aralkenyl- oder Alkenarylreste, vorteilhaft jedoch Acryl-, Methacryl-, Vinyl- oder Styryl-, und davon besonders vorteilhaft die Acryl- und Methacrylreste, die die polymerisierbaren Gruppen vieler Monomerer in Dentalmaterialien sind.

Die Aldehyd-, Epoxid-, Isocyanat- oder Halotriazingruppen dieser Haftvermittler können direkt am höhermolekularen Grundgerüst, aber auch über einen aliphatischen, aromatischen oder heterocyclischen Rest an dieses gebunden sein.

So kann es sich bei den Aldehydgruppen z.B. um Formylreste, Reste von Acetaldehyd, Vanillin, Salicylaldehyd, O-Phthalaldehyd, Anisaldehyd und Furfural handeln.

Sie können auch als Acetale oder Halbacetale vorliegen.

Die Epoxidgruppen können Teile des höhermolekularen Grundgerüstes sein

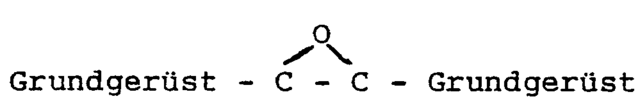

oder z.B. als Epoxyglycidyl- oder Epoxypropoxyphenylrest gebunden sein.

Sie können aber auch in Form ihres Stickstoffanalogens, nämlich in Form eines Epimins vorliegen, welches das gleiche Reaktions- und Haftverhalten wie die Epoxide aufweist. Dabei kann es sich sowohl um den

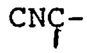

als auch um den CNN-Dreiring handeln.

Die Isocyanatgruppen sind direkt am Grundgerüst oder z.B. als Isocyanatophenyl- oder als Isocyanatoethylester gebunden.

Die Halotriazingruppen können direkt an das Gerüst oder z.B. über einen Aminoalkyl-, Hydroxyalkyl- oder einen Anilino-Rest gebunden sein.

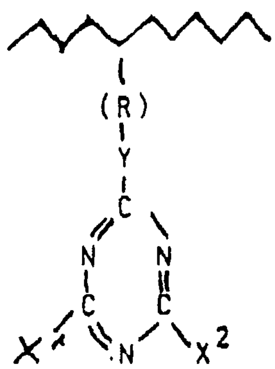

(Grundgerüst)
wobei R = kein oder beliebiger Rest
$X^1$, $X^2$=F, Cl, Br
Y = NR', O,
wobei R' = H oder Alkyl

Die oligomeren oder polymeren Grundgerüste können linearer, verzweigter oder zyklischer Natur sein.

Sie können Polymerisate von ethylenisch ungesättigten Monomeren, aber auch oligomere oder polymere Verbindungen wie z.B. Polyester, Polyamide, Polyether, Polyphosphazene und Polysaccharide sein, soweit ihr Gerüst ausreichend hydrolysestabil ist und nicht selbst mit den Haftgruppen reagiert. Die Grundgerüste können die Haftgruppen bereits beim Aufbau enthalten, es können aber auch andere funktionelle Gruppen vorhanden sein, die ein nachträgliches Aufpfropfen der Haftgruppen sowie das Anknüpfen der polymerisierbaren Gruppen erlauben.

Bevorzugte Grundgerüste sind Polymerisate aus ethylenisch ungesättigten Monomeren.

Dabei ist einerseits eine Gruppe von Monomeren geeignet, die zu Homo-oligomerisaten oder Homopolymerisaten führt, andererseits eine Gruppe, die durch Kombination verschiedener Monomerer zu Co-oligomerisaten oder Co-polymerisaten führt. Aus der Gruppe der Homopolymerisate sind z.B. Oligo- oder Polymerisate von ungesättigten Aldehyden, Epoxiden, Isocyanaten oder Halotriazinverbindungen wie z.B. Vinylformaldehyd, Styrylaldehyd, Epoxyglycidylmethacrylat, Styrylisocyanat, Allylaminodichlortriazin usw. geeignet (A):

(A) 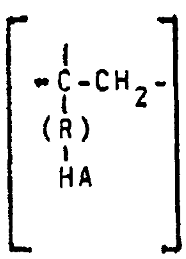

wobei R = beliebiger inerter Rest

HA = Haftgruppen:
Aldehyd, Epoxid, Isocyanat, Halotriazin.

Sie können dann in einem zweiten Schritt teilweise mit polymerisierbaren Gruppen versetzt werden, indem sie z.B. mit Hydroxyalkylmethacrylaten umgesetzt werden zu (B), (C) oder (D):

(B) 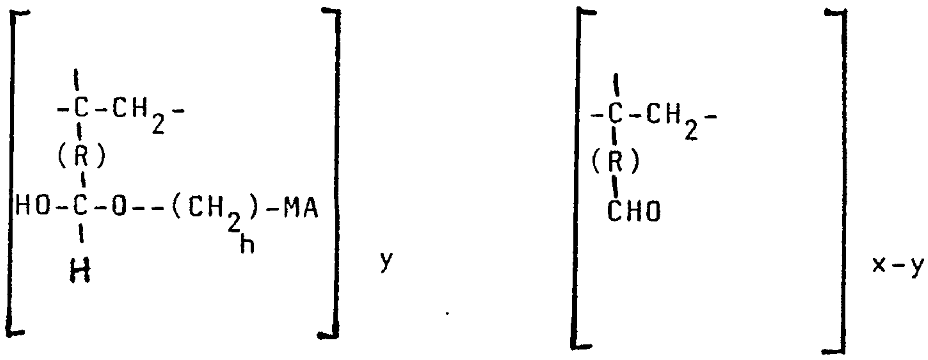

(C) 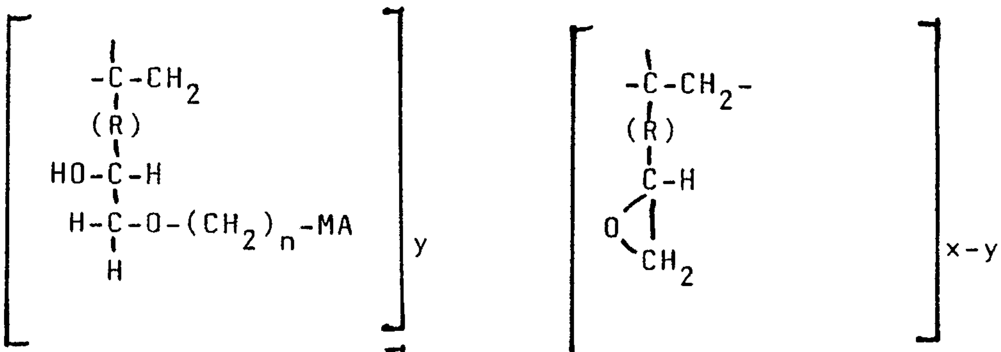

(D) 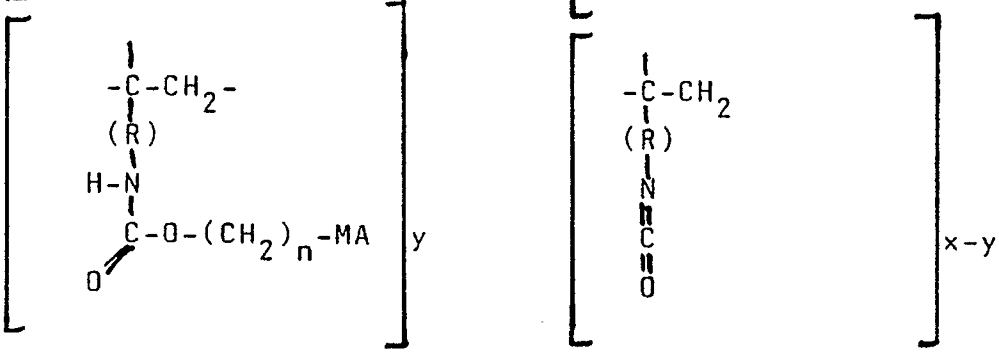

wobei MA einen Methacrylrest $-O-\overset{O}{\overset{\|}{C}}-\overset{CH_3}{\overset{|}{C}}=CH_2$ bedeuten soll.

Es kann aber auch z.B. ein Polyisocyanat durch teilweises Versetzen mit Aminoalkylaldehyd mit Aldehydgruppen und dann durch teilweises Versetzen mit Hydroxyalkylmethacrylat mit Methacrylgruppen versehen werden, so daß man z.B. polymethacrylierten Polybutyralaldehyd erhält.

Das Polyisocyanat kann z.B. auch zunächst teilweise mit Epoxypropanol und danach der Rest der Isocyanatgruppen mit Hydroxyalkylmethacrylat zu polymethacryliertem Polyglycidylapoxid umgesetzt werden.

Aus der Gruppe der Co-oligomerisate oder Co-polymerisate sind Co-oligomerisate oder Co-polymerisate von z.B. ungesättigten Isocyanaten mit ungesättigten Epoxiden oder mit ungesättigten Halotriazinverbindungen zu nennen, (E) (F):

(E) 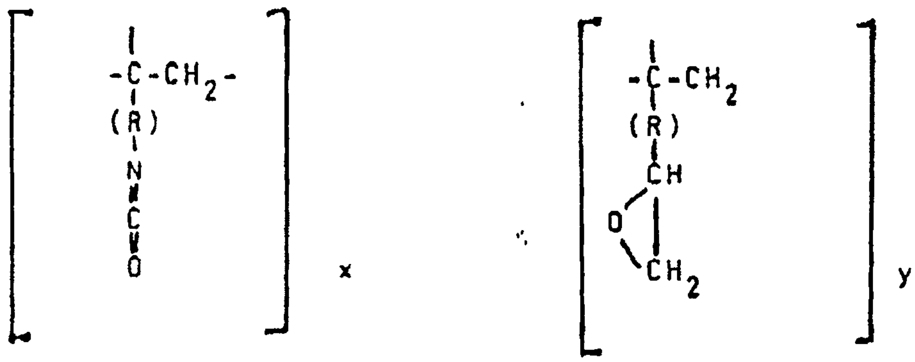

(F) 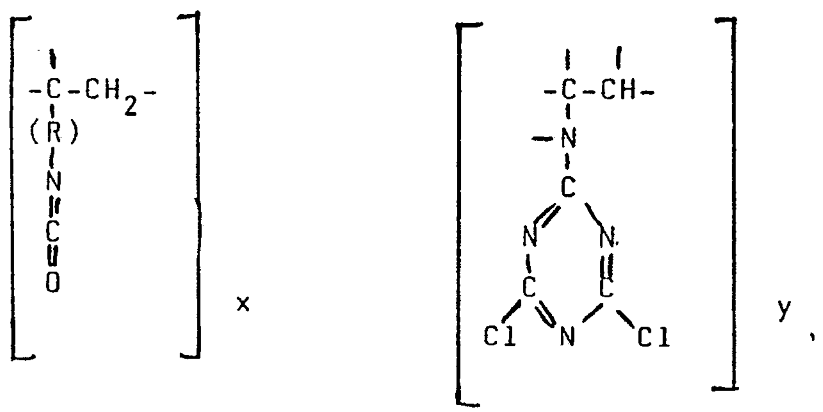

Verbindungen dieser Art können dann z.B. mit Hydroxyalkylmethacrylaten über die reaktivere Gruppe teilmethacryliert werden, so daß erfindungsgemäße Verbindungen wie (G) und (H) entstehen :

(G) 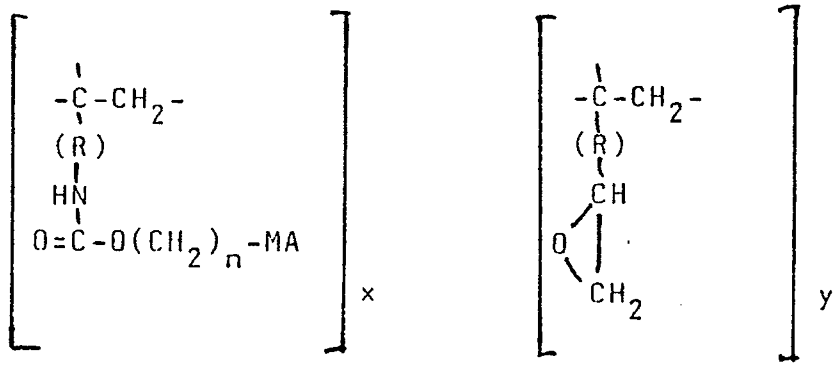

(H) 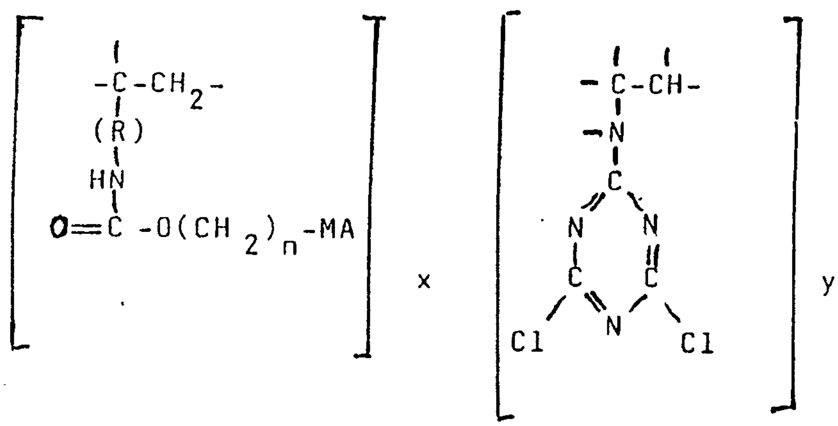

Beim Aufbau der Grundgerüste können auch Einheiten mit einpolymerisiert werden, die weder Aldehyd-, Epoxid-, Isocyanat- oder Halotriazingruppen tragen noch mit einer polymerisierbaren Gruppe versehen sind.
So kann es nützlich sein, auf diese Weise die Löslichkeit zu verändern, wie z.B. beim Einbau von inerten Methylmethacrylat-Einheiten (I):

(I) 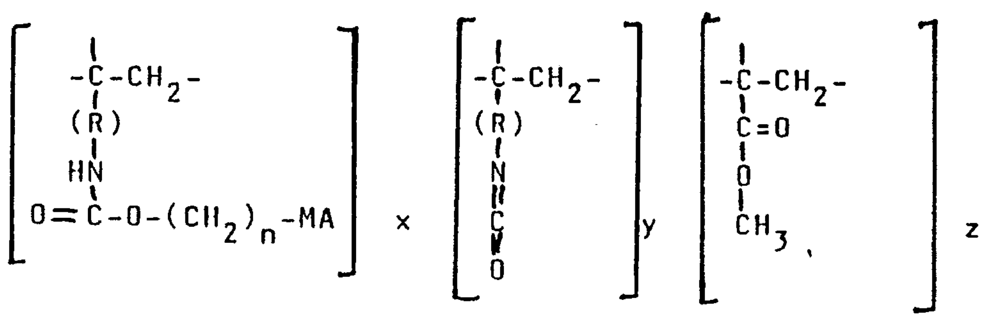

Auch der Einbau von Einheiten mit Gruppen, die Teile eines Polymerisations-Katalysatorsystems sein können, kann von Vorteil sein, wobei diese Gruppen nicht unbedingt bei der Homo- oder Co-Polymerisation des Grundgerüstes als solche Gruppen vorhanden sein müssen, sondern auch nachträglich z.B. über eine Isocyanatgruppe angeknüpft werden können.

Die Art der Reaktionsführungen, die zu den oligomeren oder prepolymeren Grundverbindungen führen, kann durch die Wahl des Lösungsmittels, der Löslichkeiten, der Konzentration, der Temperaturen und die Wahl der Polymerisationskatalysatoren bestimmt werden und sind dem Fachmann bekannt.

Wichtig kann sein, daß die benutzten Polymerisationskatalysatoren durch die Reaktion selbst zerstört werden oder eventuelle Reste von diesen vor dem Einbringen der polymerisierbaren Gruppen der erfindungsgemäßen Verbindungen entfernt werden.

Die oligomeren Haftverbindungen der erfindungsgemäß verwendeten Mischungen haben vorteilhafterweise ein Molekulargewicht von größer als 500, die prepolymeren Verbindungen haben vorteilhafterweise ein Molekulargewicht von größer als 1.500, bevorzugt nicht größer als 100.000, besonders bevorzugt nicht größer als 20.000.

Die erfindungsgemäß verwendeten Mischungen werden bevorzugt als Mischung mit anderen polymerisierbaren Verbindungen eingesetzt.

So können die Mischungen noch polymerisierbare ungesättigte Monomere, Oligomere oder Polymere enthalten, die keine Aldehyd-, Epoxid-, Isocyanat- oder Halotriazingruppen aufweisen.

Besonders eignen sich jene Monomere, die Bestandteile Üblicher polymerisierbarer Composites und polymerisierbarer Dentalharzmischungen sind und die keine Alkoholgruppen aufweisen, wie z.B Triethylenglycoldimethacrylat oder Ethoxyliertes Bisphenol-A-dimethacrylat.

Ebenso können die Mischungen Beimengungen von anderen Verbindungen mit Aldehyd-, Epoxid-, Isocyanat- oder Halotriazingruppen enthalten.

Vorteilhaft kann auch ein Zusatz von Verbindungen sein, die neben der Vernetzung über eine radikalische Polymerisation der ungesättigten Gruppen zu einer parallel laufenden Vernetzung von Aldehyd-, Epoxid-, Isocyanat- oder Halotriazingruppen führen kann. Verbindungen solcher Art sind z.B. Polyole, Polyamine, Polyamide oder Proteine. Eine solche "duale" Durchhärtung kann eine interessante Modifizierung bedeuten, die sowohl eine Haftung auf sehr polarem Substrat,- bei genügend großen Anteilen an Polyolen, Polyaminen, Polyamiden oder Proteinen sogar in wässrigem Medium bewirken, andererseits aber auch in vernetzenden Systemen auf dieser Basis zu einer Haftung zu radikalisch polymerisierenden Systemen wie z.B. zu Composites aus (Meth-) Acrylat-Basis führen kann.

Hilfsweise kann auch ein leichtflüchtiges, inertes Lösungsmittel zugesetzt sein, um einen besonders dünnen Auftrag der Mischung auf dem Gegenstand, auf dem sie haften soll, zu erreichen.

Zum Härten der erfindungegemäß verwendeten Mischungen können Polymerisationskatalysatoren zugesetzt sein, Es eignen sich prinzipiell alle diejenigen Systeme, die eine radikalische Polymerisation von olefinischen Verbindungen auslösen können.
Zusätzlich können aber auch für duales Aushärten noch Polyadditions- oder Polykondensationskatslysatoren zugegen sein.

Dabei ist es nicht wesentlich, ob die Katalysatorreaktion durch Erhitzen, durch Hinzufügen eines den Katalysator zur Reaktion bringenden Aktivators oder durch Bestrahlen mit Licht initiiert wird. Vielmehr ist es wichtig, daß des Katalysatorsystem ausreichend löslich in der Mischung ist und nicht wesentlich durch die verwendete Verbindung blockiert oder zum Zerfall gebracht wird,

Auch heilende Komponenten wie z.B. Cortison oder Cortikoide, Klauenöl oder andere können zugegeben werden, sind dann aber aus rein medizinischen Gründen indiziert, Aus ähnlichén Gründen können auch Fluorid spendende Verbindungen, wie z.B. Natriumfluorphoaphat oder Aminfluoride, zugesetzt sein.

Die erfindungsgemäß verwendeten Mischungen gen können auch mit gegebenenfalls oberflächenbehandelten anorganischen oder organischen Füllstoffen versetzt sein, wobei der anorganische Füllstoff aus der Gruppe der für Composites üblichen Mehle von Quarz, mikrofeiner Kieselsäure, Aluminiumoxid, Bariumgläsern und anderen mineralischen (inerten) Subatanzen, möglichst in silanisierter Form, sein kann, aber auch aus der für Zemente üblichen Gruppe von feinteiligen Füllstoffen wie Pulver von Metallhydroxiden, Metalloxiden, Gläsern und Keramiken, Zeolithen, Unedelmetallen und Apatit.

Die erfindungsgemäß verwendeten Mischungen können leicht durch Variationen der Verhältnisse von polymerisierbaren Gruppen zu haftenden Aldehyd-, Epoxid-, Isocyanat- oder Halotriazingruppen oder auch durch die Wahl der Molekülgröße in ihren Eigenschaften modifiziert werden.

Für sich allein oder in Mischungen mit anderen polymerisier-baren Verbindungen können sie mit verschiedensten Füllstoffen gefüllte, nach der Aushärtung mechanisch sehr feste und auf verschiedensten Untergründen haftende Werkstoffe sein. Selbst bei Gehalten von 1 bis 10% an Haftverbindungen zeigen diese Mischungen, gefüllt oder ungefüllt, nach der Polymerisation eine deutliche Haftwirkung oder Haftverbesserung auf biologischen Substraten sowie einen sehr festen Verbund mit Composites, sofern diese kurz devor polymerisiert oder kurz danach aufpolymerisiert sind.

Besonders für die Haftung an Zahn- und Knochensubstanz ergeben sich bessere Möglichkeiten von sicherer enzuwendenden Haftmischungen und dauerhafteren festeren Verbindungen von Versiegelungs- und Füllungsmaterialien zu denselben und als Kleber zwischen diesen Substraten.

So können die erfindungsgemäß verwendeten Mischungen als haftende Dentin-, Schmelz- oder Knochenadhäsive, als Versiegelungsmaterialien für Schmelz, Fissuren oder Zahnhalsdefekte, als gut haftende Schutzlacke bei Überempfindlichkeit des Zahnhalses, als Wurzelkanalfüllungsmaterialien, Kavitätenliner oder Kronen- und Brückenzemente und als Zement für orthodontische Zwecke aber auch als Knochenzement sehr nützlich sein.
Auch wenn sich die Beschreibung der Erfindung im wesentlichen auf die Anwendung auf Zahn- oder

Knochensubstanz und besonders auf die Reaktion mit collagenhaltigen Anteilen dieser Substanzen sowie auf Kunststoffe, die Polymerisate von Monomeren mit ungesättigten Gruppen sind, bezieht, so ist die Erfindung jedoch auch auf anderen Gebieten anwendbar. Das gilt besonders für die Anwendung auf menschlichen, tierischen und pflanzlichen Geweben sowie auf Produkten aus tierischen oder pflanzlichen Geweben, wie z.B. Leder, soweit diese oder zumindest deren Oberfläche mit den Aldehyd-, Epoxid-, Isocyanat- oder Halotriazingruppen reagieren können oder diese über eine Benetzungsverbesserung eine merkliche Haftwirkung ergeben.

Dieses gilt besonders für Oberflächen, die z.B. mit natürlichen, modifizierten oder synthetischen Bioadhäsiven auf der Basis von Proteinen als sogar unter Wasser haftende Systeme - wie z.B. mit "Muschelkleber" - behandelt sind.

Nachstehende Beispiele dienen der Erläuterung der Erfindung. Wenn nichts anderes angegeben ist, beziehen sich die Teile- und %-Angaben auf das Gewicht.

Beispiel 1
Herstellung von polymethacryliertem Polyvinylformal:

4,5 g Polyvinylformal (Ega-Chemie) werden in 40 ml Tetrahydrofuran mit 2,5 g Hydroxyethylmethacrylat versetzt. Nach 4 Wochen hatte das Hydroxyethylmethacrylat vollständig reagiert.
Nach Abziehen des Tetrahydrofurans blieb ein hochviskoses braunes Öl von polymethacryliertem Polyvinylformal zurück.

Beispiel 2
Herstellung eines lichthärtenden Dentinhaftmittels

Es wird eine Mischung (I) bereitet aus
55 Teilen Bisphenol-A-glycidyldimethacrylat (BIS-GMA)
45 Teilen Triethylenglycoldimethacrylat (TEDMA)
2 Teilen polymethacrylierten Polyvinylformals aus Beispiel 1
0,3 Teilen Campherchinon und
0,3 Teilen Butyldimethylanilin

Als Vergleichsmischung (II) wurde eine ähnliche Mischung bereitet, die sich nur durch Weglassen der 2 Teile des polymethacrylierten Polyvinylformals unterschied. Beide Mischungen konnten innerhalb 10 sec durch Halogenlicht ausgehärtet werden.

Die Haftung wurde auf einer frisch polierten Rinderzahn-Dentinfläche getestet.

Es wurden mehrere Zähne sowohl mit der erfindungsgemäßen Mischung (I) als auch mit der Vergleichsmischung (II) durch zweimaliges Aufpinseln und jeweils 10 sec Lichthärten vorbereitet und darauf jeweils Zylinder (Durchmesser 4 mm, Höhe 6 mm) des dentalen Füllungsmaterials "Composite Merz lichthärtbar" (Fa.Merz, Deutschland) aufpolymerisiert.

Nach 24-stündiger Wasserlagerung bei 37° Celsius wurde die Adhesivkraft im Zugversuch mit Hilfe einer handelsüblichen Zugkraftprüfmaschine ermittelt:
Haftmischung (I) : 7,3 $N/mm^2$
Haftmischung (II) : 0,4 $N/mm^2$

Auch eine Vergleichsmischung in der das polymethacrylierte Polyvinylformal durch die gleiche Menge (2 Teile) Hydroxybenzaldehydmethacrylat ersetzt ist und deren Haftvermögen am Rinderdentin auf gleiche Weise ermittelt wurde, zeigte nur 1,8 $N/mm^2$.

Beispiel 3
Herstellung von polymethacryliertem Polystyrylisocyanat:

10,5 g Poly [methylene (polyphenylisocyanat)] (Aldrichchemical Comp.) mit einem Molekulargewicht von 360 wurden mit 10,5 g Hydroxyethylmethacrylat versetzt und gerührt. Nach 24 Stunden ist im IR-Spectrum die Intensität der N = C = O Bande bei 2265 $cm^{-1}$ deutlich geringer geworden, chromatographisch ist kein Hydroxyethylmethacrylat mehr nachzuweisen. Das resultierende etwas rötliche, zähe Öl erweist sich als polymethacryliertes Polystyrylisocyanat.

Beispiel 4
Herstellung einer auf Zahnsubstanz gut haftenden 2-Komponenten-Mischung

Es wird eine Aktivator-Komponente hergestellt aus
55 Teilen ethoxiliertes Bisphenol-A-dimethacrylat (Bis-EMA)
45 Teilen TEDMA
2 Teilen Butyldimethylanilin
10 Teilen polymethacryliertem Polystyrylisocyanat aus Beispiel 3

Eine Katalysator-Komponente wird hergestellt aus
55 Teilen Bis-GMA
45 Teilen TEDMA
1 Teil Benzoylperoxid
0,04 Teilen Butyliertes Hydroxytoluol

Eine Mischung von gleichen Teilen dieser beiden Komponenten härtet innerhalb von 1 min. aus.

Es werden Proben von Rinderzähnen sowohl im Dentinbereich als auch im Schmelzbereich für die Ermittlung der Haftfestigkeit vorbereitet wie in Beispiel 2 und darauf Composite Merz ausgehärtet.
Nach 24-stündiger Lagerung in Wasser von 37° Celsius wurde die Adhesivkraft mit Hilfe einer Zugprüfmaschine ermittelt:
Haftung am Dentin : 8,9 $N/mm^2$
Haftung am Schmelz : 13,4 $N/mm^2$

Beispiel 5
Herstellung eines lichthärtenden Klebers:

Es wird eine lichthärtende Mischung hergestellt aus
100 Teilen Bis-EMA
100 Teilen TEDMA
100 Teilen polymethacryliertem Polystyrylisocyanat aus Beispiel 3
1 Teil Campferchinon und
1 Teil Butyldimethylanilin

Es werden Proben von Gold, Unedelmetall (Resilloy) und Porzellan eingeschliffen, poliert und mit Aceton gewaschen. Auf die Metallflächen wurde 2 x eine dünne Schicht des lichthärtenden Klebers gegeben und zusammen mit Zylindern von "Composite-Merz, lichthärtbar" (Fa.Merz) ausgehärtet. Nach 2 Stunden wurde die Haftfestigkeit im Zugversuch ermittelt.

| | |
|---|---|
| Gold | 4,7 $N/mm^2$ |
| Unedelmetall | 11,5 $N/mm^2$ |
| Porzellan | 18,0 $N/mm^2$ |

Beispiel 6
Haftprimer für ein Bracket-Adhesiv.

Ein Haftprimer (Aktivatorkomponente) wird hergestellt aus
45 Teilen Bis-EMA
37 Teilen TEDMA
10 Teilen methacryliertes Polystyrylisocyanat aus Beispiel 3
+ 2 Teilen Butyldimethylanilin

Für orthodontische Zwecke werden Metallbrackets (mit Gitter-Retentionen) zum einen Teil mit dem erfindungsgemäßen Haftprimer, ein anderer Teil mit dem Primer des Primer/Paste-Bracket Adhesivs "Right-On No Mix Adhesive", (TP Laboratories, USA) dünn bestrichen.
Ebenso wurde Rinderschmelz einerseits sowohl mit dem erfindungsgemäßen, als auch mit dem "Right-On" - Primer versehen. Dann wurde jeweils zwischen Schmelz und Metall die Paste (Katalysator-Komponente) des "Right-On" Produktes gegeben,und die erfindungsgemäß behandelten und die handelsüblich behandelten Körper für 40 sec. fest zusammengedrückt. Alle Proben wurden schnell hart und klebten.
Nach 2 Stunden wurden die Brackets vom Schmelz abgerissen. Bei den Proben mit dem erfindungsgemäßen Primer wurde ein Abriss zwischen Metall und Kleber, beim Produkt "Right-On" ein Abriss zwischen Zahn und Kleber beobachtet.

Damit kann ein Bracket-Adhesiv mit erfindungsgemäßem Primer im Gegensatz zu dem Produkt "Right-On" ohne schmelzzerstörendes Ätzverfahren angewandt werden.

**Patentansprüche**

1. Verwendung von polymerisierbaren Mischungen zur Herstellung von Adhäsivprodukten für biologische Substrate, dadurch gekennzeichnet, daß die Mischungen als Haftvermittler oligomere oder prepolymere organische Verbindungen enthalten, die neben mehreren polymerisierbaren ungesättigten Gruppen als funktionelle Haftgruppen aus-schließlich mehrere Aldehyd-, Epoxid-, Isocyanat- oder Halotriazingruppen an ein oligomeres oder polymeres Grundgerüst gebunden enthalten.

2. Verwendung nach Anspruch 1, dadurch gekennzeichnet, daß die Haftvermittler mindestens drei polymerisierbare ungesättigte Gruppen und mindestens drei Aldehyd-, Epoxid-, Isocyanat- oder Halotriazingruppen gebunden enthalten.

3. Verwendung nach einem der Ansprüche 1 oder 2, dadurch gekennzeichnet, daß die polymerisierbaren ungesättigten Gruppen Acryl-, Methacryl-, Vinyl-, Styryl- oder eine Mischung derselben bedeuten.

4. Verwendung nach einem der Ansprüche 1-3, dadurch gekennzeichnet, daß die Aldehydgruppe geschützt in Form eines Acetals oder Halbacetals vorliegt.

5. Verwendung nach einem der Ansprüche 1-4, dadurch gekennzeichnet, daß die Epoxidgruppe in Form eines Epimins vorliegt.

6. Verwendung nach einem der Ansprüche 1-4, dadurch gekennzeichnet, daß die Halotriazingruppe eine 4,6-Dichlor-1,3,5-triazingruppe ist.

7. Verwendung nach einem der Ansprüche 1-6, dadurch gekennzeichnet, daß die oligomeren oder prepolymeren Grundgerüste Homo- oder Copolymerisate von ethylenisch ungesättigten Monomeren sind.

8. Verwendung nach einem der Ansprüche 1-6, dadurch gekennzeichnet, daß die oligomeren oder polymeren Grundgerüste Polyester, Polyamide, Polyether, Polysulfone, Polyphosphazene oder Polysaccharide sind.

9. Verwendung nach einem der Ansprüche 1-8, dadurch gekennzeichnet, daß die Haftvermittler ein Molekulargewicht von mindestens 500 aufweisen.

10. Verwendung nach einem der Ansprüche 1-8, dadurch gekennzeichnet, daß die Haftvermittler ein Molekulargewicht von mindestens 1.500 aufweisen.

11. Verwendung nach einem der Ansprüche 1-10, dadurch gekennzeichnet, daß die Haftvermittler ein Molekulargewicht von maximal 100.000 aufweisen.

12. Verwendung nach einem der Ansprüche 1-10, dadurch gekennzeichnet, daß die Haftvermittler ein Molekulargewicht von maximal 20.000 aufweisen.

13. Verwendung nach einem der Ansprüche 1-12, dadurch gekennzeichnet, daß die Mischungen poly(meth)acryliertes Polyvinylformal enthalten.

14. Verwendung nach einem der Ansprüche 1-12, dadurch gekennzeichnet, daß die Mischungen poly(meth)acryliertes Polystyrylurethanglycidylepoxid enthalten.

15. Verwendung nach einem der Ansprüche 1-12, dadurch gekennzeichnet, daß die Mischungen poly(meth)acryliertes Polystyrylisocyanat enthalten.

16. Verwendung nach einem der Ansprüche 1-12, dadurch gekennzeichnet, daß die Mischungen poly(meth)acryliertes Polyallylaminodichlortriazin enthalten.

17. Verwendung nach einem der Ansprüche 1-16, dadurch gekennzeichnet, daß die Mischung zusätzlich andere polymerisierbare ungesättigte Verbindungen enthält.

18. Verwendung nach einem der Ansprüche 1-17, dadurch gekennzeichnet, daß die Mischungen zusätzlich

Verbindungen mit Aldehyd-, Epoxid-, Isocyanat- oder Halotriazingruppen enthalten.

**19.** Verwendung nach einem der Ansprüche 1-18, dadurch gekennzeichnet, daß die Mischungen zusätzlich Verbindungen enthalten, die Mischungsbestandteile mit Aldehyd-, Epoxid-, Isocyanat- oder Halotriazinggruppen vernetzen können.

**20.** Verwendung nach Anspruch 19, dadurch gekennzeichnet, daß diese Verbindungen Polyole, Polyamine, Polyamide oder Proteine sind.

**21.** Verwendung nach einem der Ansprüche 1-20, dadurch gekennzeichnet, daß die Mischung zusätzlich ein Lösungsmittel enthält.

**22.** Verwendung nach einem der Ansprüche 1-21, dadurch gekennzeichnet, daß die Mischungen zusätzlich Polymerisations-, Polyadditions- oder Polykondensationskatalysatoren enthalten.

**23.** Verwendung nach einem der Ansprüche 1-22, dadurch gekennzeichnet, daß die Mischung gegebenenfalls oberflächenbehandelten, anorganischen oder organischen Füllstoff enthält.

**24.** Verwendung nach Anspruch 1-23, dadurch gekennzeichnet, daß das Adhäsivprodukt ein Versiegelungs- oder Füllungsmaterial für ein biologisches Substrat ist.

**25.** Verwendung von Mischungen nach einem der Ansprüche 1-23 zur Herstellung eines Adhäsivproduktes für eine haftvermittelnde polymerisierbare Schicht zwischen einem biologischen Substrat und einem polymerisierbaren Kunststoffmaterial auf der Basis von Verbindungen mit ungesättigten Gruppen.

**26.** Verwendung von polymerisierbaren Mischungen nach einem der Ansprüche 1-23 zur Herstellung eines Adhäsivproduktes für einen Klebstoff für biologische Substrate.

**27.** Verwendung nach einem der Ansprüche 1-26, dadurch gekennzeichnet, daß das biologische Substrat Zahn- oder Knochensubstanz ist.

**28.** Verwendung nach einem der Ansprüche 1-26 zur Herstellung eines Mittels für dentale Zwecke.

## Claims

**1.** The use of polymerisable compositions for the preparation of adhesive products for biological substrates, characterised in that the compositions contain, as bonding agents, oligomeric or prepolymeric organic compounds which in addition to containing several polymerisable unsaturated groups, contain bonding groups consisting exclusively of several aldehyde, epoxide, isocyanate or halotriazine groups attached to an oligomeric or polymeric basic structure.

**2.** Use according to Claim 1, characterised in that the bonding agents contain at least three polymerisable unsaturated groups and at least three aldehyde, epoxide, isocyanate or halotriazine groups.

**3.** Use according to one of the Claims 1 or 2, characterised in that the polymerisable unsaturated groups are acrylic, methacrylic, vinyl or styryl groups or a mixture thereof.

**4.** Use according to one of the Claims 1-3, characterised in that the aldehyde group is protected in the form of an acetal or semi-acetal.

**5.** Use according to one of the Claims 1-4, characterised in that the epoxide group is in the form of an epimine.

**6.** Use according to one of the Claims 1-4, characterised in that the halotriazine group is a 4,6-dichloro-1,3,5-triazine group.

**7.** Use according to one of the Claims 1-6, characterised in that the oligomeric or prepolymeric basic structures are homo- or copolymers of ethylenically unsaturated monomers.

8. Use according to one of the Claims 1-6, characterised in that the oligomeric or polymeric basic structures are polyesters, polyamides, polyethers, polysulphones, polyphosphazenes or polysaccharides.

9. Use according to one of the Claims 1-8, characterised in that the bonding agents have a molecular weight of at least 500.

10. Use according to one of the Claims 1-8, characterised in that the bonding agents have a molecular weight of at least 1500.

11. Use according to one of the Claims 1-10, characterised in that the bonding agents have a molecular weight of at most 100,000.

12. Use according to one of the Claims 1-10, characterised in that the bonding agents have a molecular weight of at most 20,000.

13. Use according to one of the Claims 1-12, characterised in that the compositions contain poly(meth)-acrylated polyvinylformal.

14. Use according to one of the Claims 1-12, characterised in that the compositions contain poly(meth)-acrylated polystyryl urethane glycidyl epoxide.

15. Use according to one of the Claims 1-12, characterised in that the compositions contain poly(meth)-acrylated polystyryl isocyanate.

16. Use according to one of the Claims 1-12, characterised in that the compositions contain poly(meth)-acrylated polyallyl amino dichlorotriazine.

17. Use according to one of the Claims 1-16, characterised in that the composition in addition contains other polymerisable unsaturated compounds.

18. Use according to one of the Claims 1-17, characterised in that the compositions in addition contain compounds having aldehyde, epoxide, isocyanate or halotriazine groups.

19. Use according to one of the Claims 1-18, characterised in that the compositions in addition contain compounds capable of cross-linking components of the mixture having aldehyde, epoxide, isocyanate or halotriazine groups.

20. Use according to Claim 19, characterised in that these compounds are polyols, polyamines, polyamides or proteins.

21. Use according to one of the Claims 1-20, characterised in that the composition in addition contains a solvent.

22. Use according to one of the Claims 1-21, characterised in that the composition in addition contain polymerisation, polyaddition or polycondensation catalysts.

23. Use according to one of the Claims 1-22, characterised in that the composition optionally contains surface treated, inorganic or organic filler.

24. Use according to Claims 1-23, characterised in that the adhesive product is a sealing or filling material for a biological substrate.

25. Use of compositions according to one of the Claims 1-23 for the preparation of an adhesive product for a bonding polymerisable layer between a biological substrate and a polymerisable synthetic resin material based on compounds having unsaturated groups.

26. Use of polymerisable compositions according to one of the Claims 1-23 for the preparation of an

adhesive product for an adhesive for biological substrates.

27. Use according to one of the Claims 1-26, characterised in that the biological substrate is a tooth or bone substance.

28. Use according to one of the Claims 1-26 for the preparation of an agent for dental purposes.

**Revendications**

1. Utilisation de mélanges polymérisables pour la préparation de produits adhésifs pour substrats biologiques, caractérisée en ce que les mélanges contiennent comme agents adhérents des composés organiques oligomères ou prépolymères qui, en plus de plusieurs groupes insaturés polymérisables, contiennent exclusivement, comme groupes de fixation fonctionnels adhérents, plusieurs groupes aldhéyde, époxyde, isocyanate ou halotriazine liés à un squelette oligomère ou polymère.

2. Utilisation selon la revendication 1, caractérisée en ce que les agents adhérents contiennent au moins trois groupes insaturés polymérisables et au moins trois groupes aldéhyde, époxyde, isocyanate ou halotriazine liés.

3. Utilisation selon la revendication 1 ou 2, caractérisée en ce que les groupes insaturés polymérisables sont des groupes acryle, méthacryle, vinyle, styryle ou un mélange de ceux-ci.

4. Utilisation selon l'une des revendications 1 à 3, caractérisée en ce que 1e groupe aldéhyde est présent à l'état protégé sous la forme d'un acétal ou d'un hémi-acétal.

5. Utilisation selon l'une des revendications 1 à 4, caractérisée en ce que le groupe époxyde est présent sous la forme d'une épimine.

6. Utilisation selon l'une des revendications 1 à 4, caractérisée en ce que le groupe halotriazine est un groupe 4,6-dichloro-1,3,5-triazine.

7. Utilisation selon l'une des revendications 1 à 6, caractérisée en ce que les squelettes oligomères ou prépolymères sont des homo- ou co-polymères de monomères à insaturation éthylénique.

8. Utilisation selon l'une des revendications 1 à 6, caractérisée en ce que les squelettes oligomères ou polymères sont des polyesters, des polyamides, des polyéthers, des polysulfones, des polyphosphazènes ou des polysaccharides.

9. Utilisation selon l'une des revendications 1 à 8, caractérisée en ce que les agents adhérents présentent une masse moléculaire d'au moins 500.

10. Utilisation selon l'une des revendications 1 à 8, caractérisée en ce que les agents adhérents présentent une masse moléculaire d'au moins 1500.

11. Utilisation selon l'une des revendications 1 à 10, caractérisée en ce que les agents adhérents présentent une masse moléculaire de 100.000 au maximum.

12. Utilisation selon l'une des revendications 1 à 10, caractérisée en ce que les agents adhérents présentent une masse moléculaire de 20.000 au maximum.

13. Utilisation selon l'une des revendications 1 à 12, caractérisée en ce que les mélanges contiennent du formal polyvinylique poly(méth)acrylé.

14. Utilisation selon l'une des revendications 1 à 12, caractérisée en ce que les mélanges contiennent un polystyryluréthaneglycidylépoxyde poly(méth)acrylé.

15. Utilisation selon l'une des revendications 1 à 12, caractérisée en ce que les mélanges contiennent un polystyrylisocyanate de styryle poly(méth)acrylé.

**16.** Utilisation selon l'une des revendications 1 à 12, caractérisée en ce que les mélanges contiennent une polyallylaminodichlorotriazine poly(méth)acrylée.

**17.** Utilisation selon l'une des revendications 1 à 16, caractérisée en ce que le mélange contient en outre d'autres composés insaturés polymérisables.

**18.** Utilisation selon l'une des revendications 1 à 17, caractérisée en ce que les mélanges contiennent en outre des composés avec des groupes aldéhyde, époxyde, isocyanate ou halotriazine.

**19.** Utilisation selon l'une des revendications 1 à 18, caractérisée en ce que les mélanges contiennent en outre des composés qui peuvent réticuler des constituants du mélange avec des groupes aldéhyde, époxyde, isocyanate ou halotriazine.

**20.** Utilisation selon la revendication 19, caractérisée en ce que ces composés sont des polyols, des polyamines, des polyamides ou des protéines.

**21.** Utilisation selon l'une des revendications 1 à 20, caractérisée en ce que le mélange contient en outre un solvant.

**22.** Utilisation selon l'une des revendications 1 à 21, caractérisée en ce que les mélanges contiennent en outre des catalyseurs de polymérisation, de polyaddition ou de polycondensation.

**23.** Utilisation selon l'une des revendications 1 à 22, caractérisée en ce que 1e mélange contient, le cas échéant, une charge minérale ou organique traitée en surface.

**24.** Utilisation selon l'une des revendications 1 à 23, caractérisée en ce que le produit adhésif est un matériau de scellement ou d'obturation pour un substrat biologique.

**25.** Utilisation de mélanges selon l'une des revendications 1 à 23, pour la préparation d'un produit adhésif pour une couche polymérisable favorisant l'adhérence entre un substrat biologique et une matière plastique polymérisable à base de composés portant des groupes insaturés.

**26.** Utilisation de mélanges polymérisables selon l'une des revendications 1 à 23, pour la préparation d'un produit adhésif pour une colle pour substrats biologiques.

**27.** Utilisation selon l'une des revendications 1 à 26, caractérisée en ce que le substrat biologique est une susbtance dentaire ou osseuse.

**28.** Utilisation selon l'une des revendications 1 à 26, pour la préparation d'un milieu pour applications dentaires.